# EUROPEAN PATENT APPLICATION

(11) **EP 2 708 289 A2**
(43) Date of publication of application: **19.03.2014**
(21) Application number: 13184700.6
(22) Date of filing: 17.09.2013
(51) Int. Cl.: B05B 12/08, B05B 12/14, B05B 15/12, A61M 35/00

(54) **Vented reservoir for a sprayer system**

(30) Priority: 17.09.2012 US 201261702188 P
(71) Applicant: Sunless, Inc., Macedonia, OH 44056 (US)
(72) Inventor: Cooper, Steven, Macedonia, OH Ohio 44056 (US); Thomason, Scott, Macedonia, OH Ohio 44056 (US)
(74) Representative: Konkonen, Tomi-Matti Juhani

(57) **Abstract**

Embodiments according to the present disclosure provide a system that includes a reservoir with an inlet and an outlet wherein the inlet is configured to be in fluid communication with a source of a skin treatment solution. At least one spray nozzle is fluidly coupled to the outlet of the reservoir and is configured to spray the skin treatment solution. A vent valve is operable to open and close based on the level of the skin treatment solution in the reservoir. When the vent valve is open, a cavity of the reservoir is exposed to at least atmospheric pressure. The vent valve may be configured to control liquid flow to the reservoir from the container for refilling the reservoir and to aid in the operation of changing containers and/or purging the system.

## Description

### PRIORITY CLAIM

This application claims priority to U.S. Provisional Patent Application No. 61/702,188 filed on September 17, 2012, and entitled Vented Reservoir for a Sprayer System, the disclosure of which is hereby incorporated by reference.

### TECHNICAL FIELD

The present invention relates generally to skin treatment sprayer systems, and more particularly to a reservoir and vent system for the delivery of skin treatment solutions.

### BACKGROUND

Booth spray systems for the application of skin lotions and cosmetics dispense a selectable variety of skin treatments including moisturizer and tanning treatments. Salon spray booths for sunless tanning and skin treatments offer multiple spray sessions with selections from a wide variety of skin lotions and tanning products. Many of the booth systems have moving gantries that apply the spray evenly over the full body or can be user-programmed to apply only to the face or legs. Some booths are outfitted with booth pre-heaters and full body drying systems. Automated booth spray systems used in salons consist basically of a booth structure that is either fully or partially enclosed with single or multiple spray nozzles positioned inside the booth. Reference is made to the following references generally directed to booth-type spray systems, the disclosures of which are hereby incorporated by reference: U.S. Patent No. 6,199,557 to Laughlin filed on April 19, 1999; U.S. Patent No. 7,004,407 to Cooper filed on December 4, 2002; U.S. Patent No. 7,886,684 to Cooper et al. filed on April 28, 2006; U.S. Patent No. 8,201,288 to Thomason et al. filed on August 24, 2009; U.S. Patent Application Publication No. 2010/0266776 by Cooper et al. filed on April 20, 2010; and U.S. Patent Application Publication No. 2011/0133004 by Thomason et al. filed on October 22, 2010.

The spray session is activated by the person receiving the spray treatment within the enclosure. An exhaust fan may be used to prevent overspray inside the booth or drifting spray escaping from the booth. Other booth features may include lights, voice prompts in different languages, heaters, skin drying systems, and interior washing and rinsing systems.

The electrical and mechanical components in the rinse, drain, spray, gantry, heat and exhaust systems of these automated spray booths are operated in a sequence during a spray session by a microprocessor based or other sequential controller with a manual input device such as a keypad or button panel. Spray session parameters such as liquid flow, duration of spray, heater temperature, and the like are set and adjusted by input to the controller. Salon personnel and/or consumers using the spray system may manually enter certain operation parameters for each spray session.

The spray solution used for sunless tanning is generally a water-based mixture of DHA (dihydroxyacetone) and/or erythrulose and various other skin care ingredients such as aloe vera. Often a cosmetic bronzer is added along with pleasant scents and other ingredients to enhance the tanning results and experience, such as formulations to balance skin pH. For best results, the spraying of the solution utilizes a finely atomized spray (mist), as opposed to using a spray stream or large spray droplets, because the mist of solution provides even coverage and reduces the risk of streaking or running of the spray deposit.

The spray systems of these booth-type skin treatment sprayers generally include single or multiple containers or tanks containing liquid spray solution which is fed to the spray nozzles by single or multiple pumps or other methods, such as gravity or Venturi. Flow is generally controlled by solenoid valves and a mechanical pump for which flow rate can be varied by varying motor speed or pressure. Multiple nozzles may be stationary, positioned along the interior walls of the booth, or they may be mounted to a moving gantry. A spray system disclosed in U.S. Patent No. 7,886,684 to Cooper et al., the disclosure of which is hereby incorporated by reference, utilizes a single dose cartridge tank system. This can be configured with a single nozzle that oscillates while moving on a gantry. In addition it can be operated without a mechanical pump, relying on gravity or Venturi feed to the nozzle. Multiple batch tank systems on skin care booth sprayers allow approximately 30 to more than 100 sessions between changing containers or re-filling the tanks. Booths with multiple tanks have the advantage of allowing a sequence of spray sessions with a choice of various lotions applied one after the other; for instance a moisturizer treatment may be applied after a tanning treatment, or a skin pH balancing spray may be applied before a tanning spray. Some booth models use refillable multiple tank systems with 2, 3 or 4 tanks. Many booth spray systems accommodate a more convenient bag-in-box system where multiple refillable and/or replaceable containers are received in a bay drawer of the unit as disclosed by U.S. Patent No. 8,201,288 to Thomason et al. filed on August 24, 2009, the disclosure of which is hereby incorporated by reference.

It is sometimes difficult to determine the quantity of the solution remaining in the containers of skin treatment solution. As such, an operator may remove and replace the container before it is fully depleted resulting in waste of skin treatment solution. Alternatively, an operator may misjudge the remaining contents of the container and the spray tanning booth may be attempting to draw from an empty container during a spray tanning session.

### SUMMARY

Embodiments according to the present disclosure provide a system for spraying a skin treatment solution. The system includes a reservoir with an inlet and an outlet wherein the inlet is configured to be in fluid communication with a source of a skin treatment solution. At least one spray nozzle is fluidly coupled to the outlet of the reservoir and is configured to spray the skin treatment solution. A vent valve is operable to open and close based on the level of the skin treatment solution in the reservoir. When the vent valve is open, a cavity of the reservoir is exposed to at least atmospheric pressure.

In a certain embodiment, the vent valve is operable to open the vent when the skin treatment solution in the reservoir drops to a predetermined level. Opening the vent allows the skin treatment solution to flow from the source to the reservoir.

In another embodiment of the present disclosure, there is provided a system for spraying a skin treatment solution where spray nozzles are controlled to operate at periodic intervals, such as during a skin spray treatment session. A vent valve is operable to expose a cavity of the reservoir to at least atmospheric pressure and to be closed to prevent the skin treatment solution in the system from exposure to ambient air. A liquid level sensor is operable to detect a level of the liquid in the reservoir, and a vent valve receives a signal from a controller causing it to open or close the vent, which may be directly or indirectly based on the detection of the level of the skin treatment solution in the reservoir.

The vent valve may be configured to enable liquid flow to the reservoir from the container for refilling the reservoir and to aid in the operation of changing containers and/or purging the system. Multiple containers and corresponding reservoir systems may be used for systems that deliver multiple spray treatments. Such spray treatments may be of spray tanning solution, moisturizer, skin preparation treatment (for example to accelerate the tan), or a spa skin treatment (for example anti-aging spray).

Technical advantages of the embodiments disclosed herein include a reservoir configured to hold a reserve amount of skin treatment solution that may be continued to be used by the spray system even after depleting a removable container. In addition, in normal operation the reservoir system may be closed from exposure to the atmosphere to protect delicate solutions that might degrade if subjected to such exposure.

Additional technical advantages include a sprayer system that prevents the build-up of negative pressure in a fluid system and continues to allow siphoning of liquid from the reservoir and delivery of that liquid to one or more spray nozzles. In addition, the sprayer system employing the vented reservoir of the present disclosure may prevent unwanted siphoning of liquid from a full removable container that is fluidly coupled to the system. Rather, the container may be fluidly coupled to the system while the vent valve is closed. In this manner, the contents of the container are prevented from flowing into the reservoir immediately upon installation. Rather, after installing the container, a user may provide input that opens the vent valve and allows the liquid to flow into the reservoir where it is available to be pumped through the conduits of the fluid system. This may prevent accidental and unwanted mixing of liquids in the fluid system, such as when a user inadvertently installs a container and thereby fluidly couples the container to a reservoir with a quantity of fluid remaining in the reservoir.

Other technical advantages will be readily apparent to one of ordinary skill in the art from the following figures, descriptions, and claims. Moreover, while specific advantages have been enumerated above, various embodiments may include all, some, or none of the enumerated advantages.

### BRIEF DESCRIPTION OF THE DRAWINGS

A more complete understanding of the invention may be obtained by reference to the following drawings:
Figures 1 schematically illustrates a reservoir and vent system adapted for spraying a skin treatment solution;
Figure 2 illustrates certain components of the reservoir and vent system of Figure 1 along with a cross-sectional view of a bag-in-box container;
Figures 3A and 3B are detailed illustrations of the components of the reservoir and vent system illustrated in Figure 2;
Figures 4 illustrates an embodiment of a skin treatment spray booth with portions broken away adaptable to employ a reservoir and vent system according to the present disclosure;
Figure 5 is a detailed illustration of the drawer shown in Figure 4 including skin treatment containers;
Figures 6 is a detailed illustration, with portions removed, of a skin treatment container of Figure 5; and
Figure 7 is a rear view of the drawer of Figures 4 and 5 showing a plurality of reservoir and vent systems according to embodiments of the present disclosure.

### DETAILED DESCRIPTION

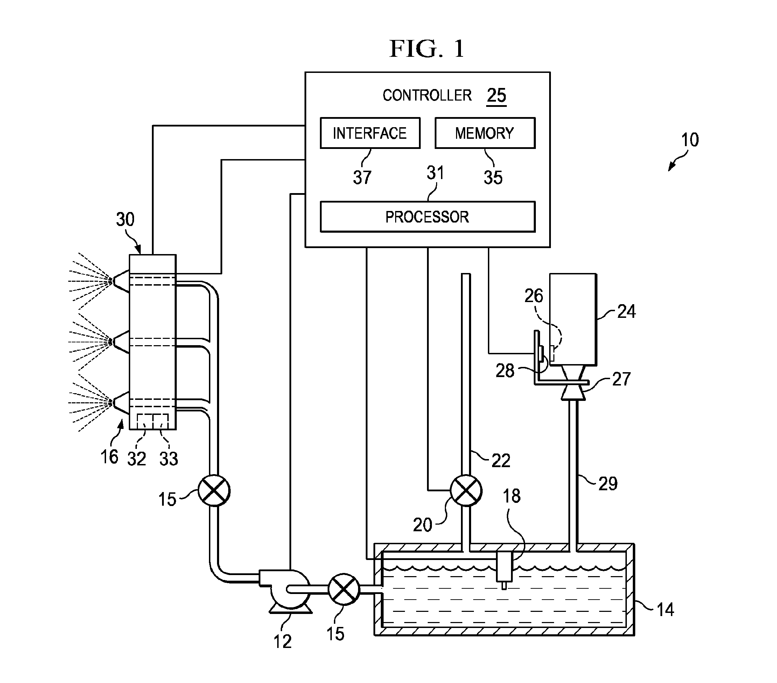

Figure 1 is a schematic illustration of a vented reservoir system 10 according to embodiments of the present disclosure. The system 10 provides a reservoir 14 that is filled from a container 24. The container 24 may be a rigid or collapsible container that is either vented or non-vented. In one embodiment, the container is a non-vented, collapsible container 24, such as a bag-in-box container that is removable from the system 10. The container 24 is disposable and replaceable with a new container, but in an alternate embodiment, the container may be refillable and reusable. The container 24 may contain a skin treatment solution to be sprayed by a sprayer system. The skin treatment solution may be a solution used in sunless tanning, such as a pre-tanning solution, a bronzer, a moisturizer, a post-tanning solution, or a solution to balance the pH of the skin. The solution may be either water or oil based. In addition, the skin treatment solution may be a skin lotion, a topical medicinal treatment, and the like.

According to an embodiment, a vent valve 20 opens or closes a vent associated with the reservoir, and thereby creates either an open or closed fluid system. According to one embodiment, the reservoir is coupled to a vent tube 22. Alternatively, the system may not include a vent tube, but rather the vent valve 20 may located at any suitable location to allow controlled venting of the reservoir 14. When the vent valve 20 is closed, the fluid system is closed and the skin treatment solution is not exposed to the atmosphere. Some types of skin treatment solution may degrade over time if exposed to the atmosphere, so a fluid system that is primarily closed allows the skin treatment solution to be effective over a longer period of time. However, in certain operations, such as filling the reservoir 14 from the container 24 and drawing solution from the reservoir 14 in certain instances, an open system facilitates such functionality. Thus, when an open system is desired as described in more detail below, the vent valve 20 is opened, the sealed cavity of the reservoir 14 is exposed to the atmospheric pressure of the ambient environment, and the reservoir 14 is vented.

Regardless of whether the fluid system is open or closed, a pump 12 draws liquid from the reservoir 14 and delivers it to a spray nozzle 16 or a plurality of spray nozzles 16. The pump 12 may be a positive displacement pump that is operable to draw liquid from the reservoir 14 and deliver the liquid to one or more spray nozzles 16. The pump 12 may be a piston or syringe pump including a linear actuator in communication with a controller 25. In an alternate embodiment, the system 10 may not include a pump or may include devices to create fluid flow in addition to the pump 12. For example, liquid may be drawn from the reservoir 14 and delivered to the nozzles 16 via a venturi effect created by the nozzles 16 themselves. As another example, an air-over liquid pressure or other gas pressure system may be employed to directly pressurize the container 24 and/or the reservoir 14. According to an embodiment, the reservoir 14 may be pressurized through its vent.

Fluid flow through the system 10 may be controlled by one or more check valves 15. For example, a check valve may be positioned in series between the reservoir 14 and the pump 12. A check valve in this location prevents unwanted fluid backflow into the reservoir 14. Alternatively or additionally, a check valve may be disposed between the pump 12 and the nozzles 16 and function as a back-up check valve such that two check valves must fail for liquid to backflow and damage certain components and cause the system to malfunction. The check valve 15 in this location prevents unwanted fluid backflow in the pump 12 (and the reservoir 14). It should be understood that embodiments of the present disclosure may employ devices other than the check valve 15 to prevent unwanted backflow. For example, in lieu of the check valve 15 may be a solenoid valve, a pump, or any other device suitable for preventing unwanted backflow in a fluid system.

Gravity, venturi, and/or the pump 12 causes the liquid to flow from the container 24 to the reservoir 14. As the pump 12 draws the liquid from the container 24 and through the reservoir 14, a vacuum created in the container 24 causes the bag to collapse as it empties. In an alternate embodiment, a rigid, non-collapsible container may be used. In this instance, the degradation of the solution may not be a concern and the non-collapsible container may be vented. However, if a rigid, non-vented container is used, liquid may be drawn from the container 24 when the vent valve 20 is open. In an alternate embodiment employing a closed system with a non-collapsible container, a container vent may be operated by the same circuit as the reservoir vent valve 20 such that the container and the reservoir 14 are vented simultaneously to allow liquid to be drawn from the container 24 into the reservoir 14. According to an alternate embodiment employing a rigid, non-vented container, the controllable vent valve 20 may be located on the rigid container or plumbed to draw air into the neck of the container through a controllable vent.

Once the container 24 is empty and flow to the reservoir 14 ceases, continuing to pump liquid through the system to the spray nozzles 16 will cause a drop of the liquid level of the reservoir 14 and a pressure differential opposing the liquid drawing functionality of the pump 12 in the closed fluid system. To alleviate this pressure differential and relieve the opposing force on the pump by equalizing the fluid pressure in the system 10, the vent valve 20 is opened. The drop in liquid level is sensed by the liquid level sensor 18 and is communicated to a controller 25, which in turn provides an opening signal to the vent valve 20, which opens the fluid system.

In addition, the controller 25 through an interface 37 can indicate that the container 24 is empty and should be replaced. Even though the removable container 24 is empty and the system has provided notice of the empty removable container 24, the reservoir 14 holds enough solution for further operation of the system without immediate replacement of the removable container. Thus, the system may have liquid sufficient for a limited number of spray cycles even though the container 24 is empty. For example, the volume of solution remaining in the reservoir 14 after the container 24 is empty (and notification is indicated) may be sufficient to allow spray cycles corresponding to one or more spray sessions including spraying the solution over the full body of the user.

The container 24 may be a bag-in-box container containing a skin treatment solution, such as a sunless tanning solution. The bag-in-box container 24 may include a machine readable tag 26. The tag 26 may be a radio frequency identification (RFID), bar code, QR (quick response) code, and the like. The tag 26 may communicate information to an interrogator 28. The interrogator 28 is in communication with the controller 25 and is operable to receive information regarding the container 24 and the solution therein and may direct control of the reservoir and vent system 10 based on that information. For example, the interrogator 28 may receive information from the tag 26 indicating that the system 10 has received a new solution in replacement of a depleted container. The controller 25 may use this information to determine that the pump 12 should be automatically calibrated and/or primed or the system purged to prepare for the new solution.

During the calibration, priming, and/or purging operations, the vent valve 20 may be open or closed. If the vent valve 20 is initially closed, the controller 25 may direct the vent valve 20 coupled to the vent tube 22 to open. The opening of the vent valve 20 exposes the fluid in the system to atmospheric pressure causing the liquid to drain from the container 24 into the reservoir 14. In this manner, a skin treatment solution may be received by the reservoir and vent system 10, and the system may be automatically prepared through the preliminary operation of priming, calibrating, and/or purging, followed by or in conjunction with filling the reservoir 14 to be operable to spray that solution through the nozzles 16. Regardless of whether the vent valve 20 is open or closed, the controller 25 may close the vent valve 24 for normal operation of the system 10.

The reservoir 14 includes the liquid level sensor 18. In certain embodiments, the liquid level sensor 18 may be a mechanical float sensor. In other embodiments, the liquid level sensor 18 may be an optical sensor or a capacitance sensor or any other sensor operable to sense a liquid level, volume, or mass in the reservoir 14. A function of the liquid level sensor 18 is to determine when the vent valve 20 should be opened or closed. For example, if the liquid level drops to a predetermined level, the liquid level sensor 18 may communicate a signal to the controller 25 directing it to cause the vent valve 20 to open. Opening the vent valve 20 relieves the pressure that may have accumulated during operation of the closed fluid system, if any. In addition, a pressure differential is created by exposing the liquid to atmospheric pressure, which causes the liquid in the container 24 to fill the reservoir 14. Once the reservoir 14 has been filled to a predetermined fill level with the solution from the container 24, the liquid level sensor 18 rises. This displacement of the sensor 18 is communicated to the controller 25, and the vent valve 20 is closed. The vent valve 24 is typically closed during normal operation, which protects any solution that might degrade or otherwise become less effective after prolonged exposure to air. In an alternate embodiment, the liquid level sensor may communicate directly with the vent valve 20, which may make the system 10 operable without the controller 25.

Motion of a gantry 30 that supports and moves the spray nozzles 16 may be directed by the controller 25. A motor 33 translates the gantry 30 linearly to any position between an upper limit position and a lower limit position to direct spray from the nozzles 16 to particular body parts of the person in the spray tanning booth. The control and movement of the gantry 30 may be coordinated by the controller 25 such that a displacement of a piston in the pump 12 correlates to a specific distance of travel of the gantry 30. For example, if the gantry 30 is to provide a skin treatment solution to just the legs of an individual standing in a spray tanning booth, a full displacement of the piston from a retracted position to an extended position is coordinated with the movement of the gantry 30 to pass over the legs of the individual. The controller 25 may also receive signals from a height sensor 32 that optically or otherwise senses the height of an individual in the spray tanning booth and can adjust the travel of the gantry 30 accordingly. Other sensors such as a time or limit switch corresponding to a particular position or motion of the gantry 30 may also be in communication with the controller 25 to provide information regarding the actual position of the gantry 30.

The controller 25 includes one or more processors 31 and memory 35. Thus, the controller is essentially a microprocessor or other logic controller in communication with at least temporary memory and functions to control the components used in the spray session and to communicate data to other components of the system 10. The controller 25 may also be in communication with an interface 37. The interface 37 may be any suitable interface that allows a human to interact with and receive information from the controller 25. In certain embodiments, the interface 37 may be a touch-screen, keypad, monitor, and the like. In a preferred embodiment, the interface 37 may be a touch-screen that allows the user to communicate with the controller 25 by touching the screen where command icons and other information are displayed. The interface 37 may also be used to program the controller 25 or provide other information to the controller 25 that may be used to operate the reservoir and vent system 10.

The controller 25 may also allow automatic or manual venting of the reservoir 14 to facilitate changing containers 24 and performing purge operations if switching to containers of different spray solutions. The vent valve 20 may be automatically or manually controlled to prevent mixing of different solutions when solutions are changed out. For example, when replacing an empty container with a full replacement container, the vent valve 20 is initially closed. Thus, even upon fluidly coupling the full container to the closed liquid system, liquid will not immediately flow from the container into the reservoir 14. Rather, the vent valve 20 remains closed until the user gives a command to the controller 25 through the interface 37 to open the vent valve 20 and allow the liquid in the container to flow into and accumulate into the reservoir 14. In this manner, liquid from the container will not flow into the reservoir and mix with any liquid remaining in the reservoir immediately upon installation of the container. A user may confirm that he has correctly installed the container in the correct container bay and there will be no unwanted mixing of the liquid in the container with any remaining liquid in the reservoir 14 before commanding to the controller to signal the vent valve 20 to open and allow the liquid to flow into the reservoir 14.

As a further example, the vented reservoir system 10 according to embodiments of the present disclosure facilitates clean replacement of an empty container 24. In replacing a container 24, some solution may remain in a coupler 27 and/or a fluid line 29. Once the empty container 24 is removed, this remaining liquid may escape the coupler 27 and leak from the container side of the coupler 27. However, by employing logic to control the vent valve 20, the liquid in the coupler 27 and the fluid line 29 can be cleared by opening the vent valve 20, which will allow gravity to cause the liquid to drop into the reservoir and prevent any unwanted leakage during replacement of the removable container.

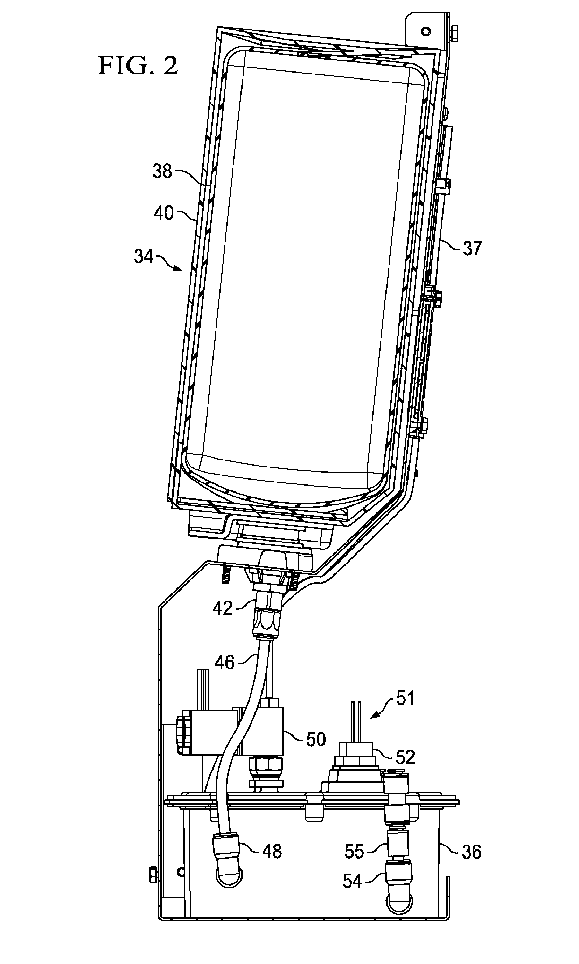

Figure 2 illustrates an embodiment of a portion of the reservoir and vent system 10 shown in Figure 1. The embodiment includes a removable container 34 fluidly coupled to a reservoir 36. The removable container 34 is shown in cross-section and is a bag-in-box type container. The removable container 34 includes a collapsible bag 38 inside a cardboard box 40. The collapsible bag 38 may be a thin-walled plastic bag suitable for containing the liquid contents of the container 34. The removable container 34 includes a specific volume of skin care solution in a removable, refillable, and disposable container. In an alternate embodiment, the removable container 34 may be formed of a rigid material, such as plastic, and may not be collapsible. A rigid container may or may not be vented.

The removable container 34 may contain any type of skin treatment solution used in the application of a cosmetic to the skin in a spraying operation. For example, the solution may be a pre-tanning solution, a moistening solution, a tanning solution, including a bronzer, or a post-tanning solution. The solution may be clear or bronze, and it may be water or oil-based.

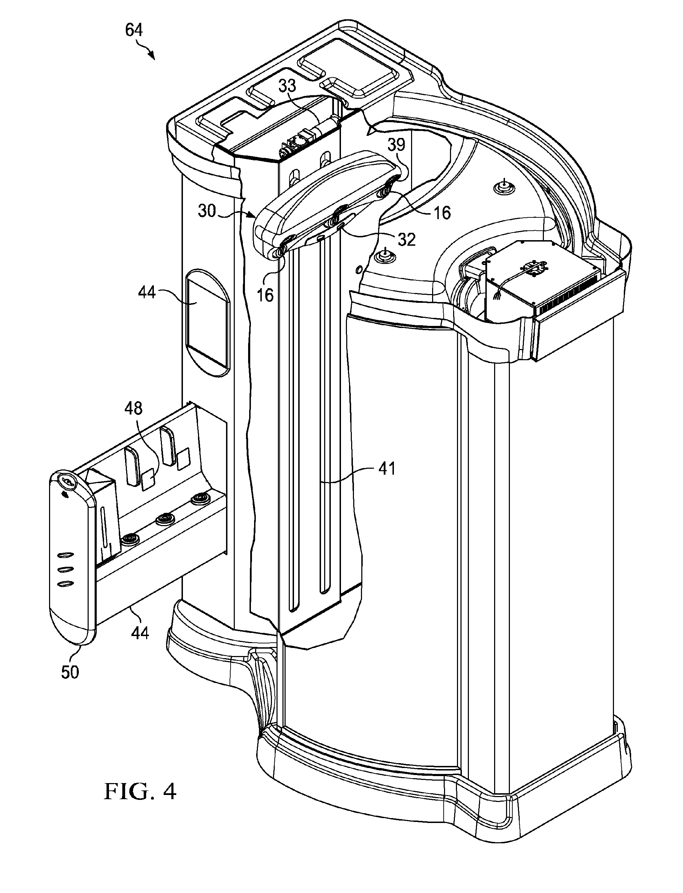

The removable container 34 includes a male fitting that may be received by a coupler 42 that is part of a spray tanning booth. More particularly, the coupler 42 may be a quick connect coupler in a retractable drawer 44 as shown in Figure 4. A solution conduit 46 is connected to the coupler 42 and runs from the coupler 42 to a reservoir inlet fitting 48 of the reservoir 36. The solution conduit 46 may be any tubing material that is suitable for allowing liquid to flow therethrough. In an alternate embodiment, the bag-in-box fitting may connect directly to the reservoir 36.

Solution flows from the removable container 38 and collects in the reservoir 36. The reservoir 36 may include a clear portion to allow an operator to visually determine a liquid level in the reservoir 36. A vent valve 50 vents the reservoir 36. According to one embodiment, the vent valve 50 is coupled to a vent tube 37 that extends from a lid of the reservoir 36 upward toward the removable container 34. The length of the vent tube 37 is such that the open end of the vent tube 37 is above the highest liquid level within the container 34. Thus, if the system is not in normal operation, the vent tube 37 can contain liquid equal to a liquid level of the removable container 34 without overflowing because the liquid level in the vent tube 37 should not rise above the liquid level in the bag-in-box container 34. Therefore, the liquid level should not be higher than the top of the open vent tube 37. The vent tube 37 may be any suitable conduit for carrying a fluid including a gas, such as air, in or out of the reservoir 36. It should be understood, the vent tube 37 is not necessary to control venting of the reservoir via the vent valve 50. Also, the function of venting the reservoir may be accomplished by fluidly coupling the reservoir to a pressure source, as opposed to the atmospheric vent described herein. The pressure source may also serve to pressurize the contents of the reservoir, and upon opening a valve, the pressure will cause the liquid in the reservoir to flow through the system to the nozzles. In this manner, liquid from the reservoir may be delivered through the nozzles without employing a pump.

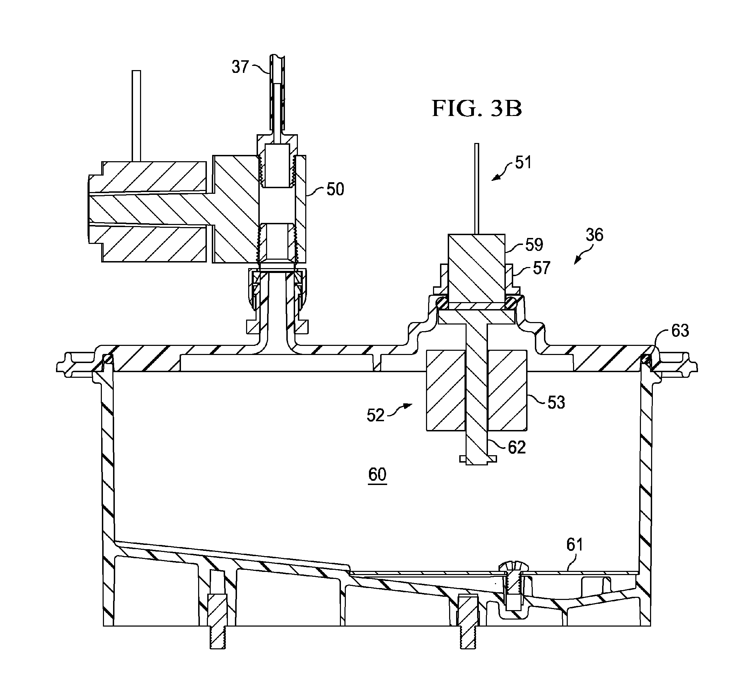

In the illustrated embodiment, the vent valve 50 is a solenoid valve. Thus, when current is supplied to the solenoid valve, it opens or closes. In other embodiments, the vent valve 50 may be a manual valve, which opens and closes based on the position of a liquid level sensor 52. A specific embodiment of a liquid level sensor 52 is shown and described with respect to Figure 3B below.

According to an alternate embodiment, the vent valve 50 may be a check valve, which is operable to vent the reservoir 36. Upon the controller actuating the pump after depleting the collapsible removable container 34, negative pressure is created in the fluid system, which will open the vent (check) valve 50. With the vent valve 50 open, the negative pressure is relieved, the reservoir 36 is vented, and the solution in the reservoir 36 can flow from the reservoir 36 where it can be pumped to the spray nozzles 16. In this embodiment, the check valve may not operate to vent the reservoir when a new removable container is installed because installing a removable container 34 does not necessarily create a negative pressure that will open the vent valve 50. Therefore, an additional valve, such as a manual bypass valve, may be disposed in the fluid system such that upon inserting a full removable container 34, the bypass valve can be opened, for example manually, to allow the solution to flow from the removable container 34 into the reservoir 36.

The reservoir 36 is in fluid communication with the pump 12 (see Figure 1). The pump 12 causes the liquid to flow from the reservoir 36, out a reservoir outlet 54, and into a conduit that runs from the reservoir 36 to one or more spray nozzles 16, as shown in Figure 1. A check valve 55 is coupled to the outlet to prevent unwanted backflow into the reservoir 36. Along the conduit, there may be any suitable number of valves to control the flow of liquid from the reservoir 36 to the spray nozzles 16.

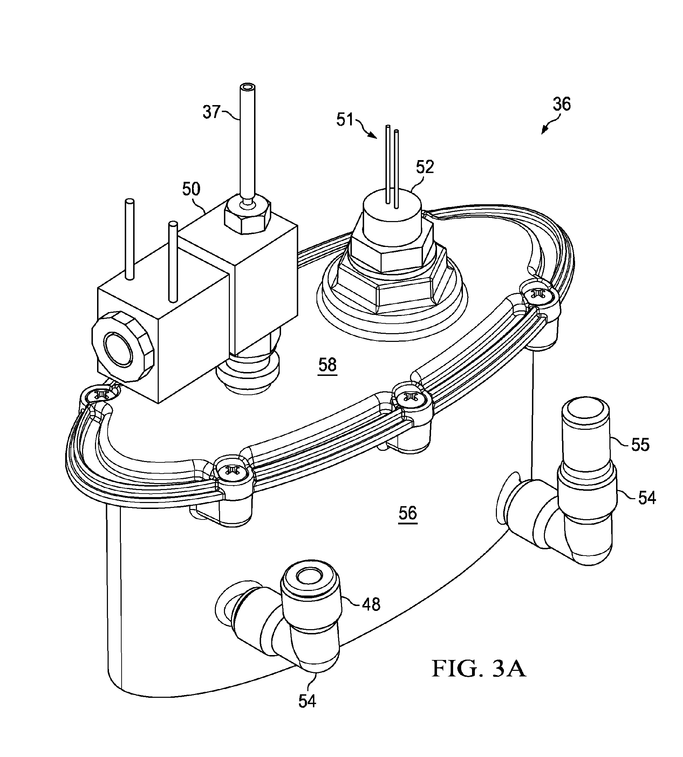

Reference is made to Figures 3A and 3B. Figure 3A is an isometric view of the vented reservoir system shown in Figure 2 without the removable container. Figure 3B is a cross-section of the reservoir and associated components shown in Figure 3A. The reservoir 36 includes a tank 56 which is covered by a lid 58. The lid 58 may be secured to the tank 56 with any suitable fastener, such as a plurality of screws. A gasket 63 is disposed between the lid 58 and the tank 56 to ensure the reservoir 36 is hermetically sealed. The lid 58 may also provide a mounting surface for the liquid level sensor 52 and the vent valve 50. In an alternate embodiment, the lid 58 may not be removable from the tank 56. However, a removable lid 58 or access ports may facilitate access to interior components of the reservoir 36, such as a filter 61 or components of the float sensor 53.

The reservoir includes a tank cavity 60 which holds a predetermined volume of solution. A filter or screen 61 may be disposed near the bottom of the cavity 60 but above the outlet. The filter prevents certain sizes of unwanted particulate from being circulated through the fluid system. A drain plug may also be disposed near the bottom portion of the cavity 60. Liquid level in the tank cavity 60 is sensed by the liquid level sensor 52, which includes a rod 62 and a float sensor 53. The float sensor 53 is vertically movable along the rod 62. The float sensor 53 will descend on the rod 62 as the liquid level decreases. When the tank cavity 60 is full, the liquid level raises the vertical position of the float sensor 53. The position of the float sensor 53 is sensed and the position, either elevated or descended, is communicated via electrical signal to the controller.

According to embodiments of the present disclosure, the descended position of the float sensor 53 indicates a predetermined level of liquid in the tank cavity 60. For example, when the liquid level in the tank cavity 60 drops to a level that causes the float sensor 53 to reach its lower limit, there may be approximately 250 ml of solution in the reservoir 36. In addition, dropping of the liquid level in the tank cavity 60 to descend the float sensor 53 also indicates that the removable container is empty because it is no longer supplying solution to the reservoir 36 to fill the reservoir and cause the float sensor 53 to be in its ascended position. In this manner, the controller receives an electrical signal through one or more electrical wires 51 running from the liquid level sensor 52 and indicates to an operator that the removable container is empty and that there is a predetermined volume of liquid in the reservoir 36 which may continue to be used. For example, an operator may be warned that the removable container needs to be replaced but that there is still enough solution in the reservoir and vent system to complete two spray tanning sessions. The predetermined volume of liquid in the reservoir may be changed by adjusting the liquid level sensor 52. According to the embodiment illustrated in Figure 3B, a jamb nut 57 that is engaged with a threaded adjustment member 59 may be used to adjust the height of the float sensor 53 such that the sensor 53 will activate at higher or lower liquid levels.

An alternate embodiment of the present disclosure may, in place of or in addition to the liquid level sensor 18, employ a float valve that when it is in its ascended state, physically blocks the reservoir from venting.

The controller 25 may direct additional actions in response to receiving a signal from the liquid level sensor 52 indicating that the liquid level in the reservoir 36 has dropped. For example, the controller may direct the vent valve 50 to open and allow the pump 12 to continue to siphon solution from the reservoir without building a negative pressure in the fluid system that opposes the siphoning action of the pump 12. In an alternate embodiment, the liquid level sensor 52 may communicate directly with a visual and/or audible signal device to indicate to an operator that the solution level in the reservoir 36 is low and the container 34 is empty. In order to minimize exposure of the solution to air, if the pump 12 is not pumping, the vent valve 50 may be closed.

Reference is made to Figure 4 which shows an isometric view of a tanning booth 64 with portions broken away to reveal internal components. Specifically, a vertically movable gantry 30 supports the plurality of nozzles 16, which in the illustrated embodiment includes three spray nozzles 16. In addition, the gantry 30 supports an auxiliary heated air outlet 39 above each nozzle 16. The heated air outlets 39 may be used in drying passes and/or may be used to heat the spray cloud emitted by the nozzles 16. A motor 33 moves the gantry 30 vertically along the tracks 41. The gantry 30 also supports the optical height sensor 32.

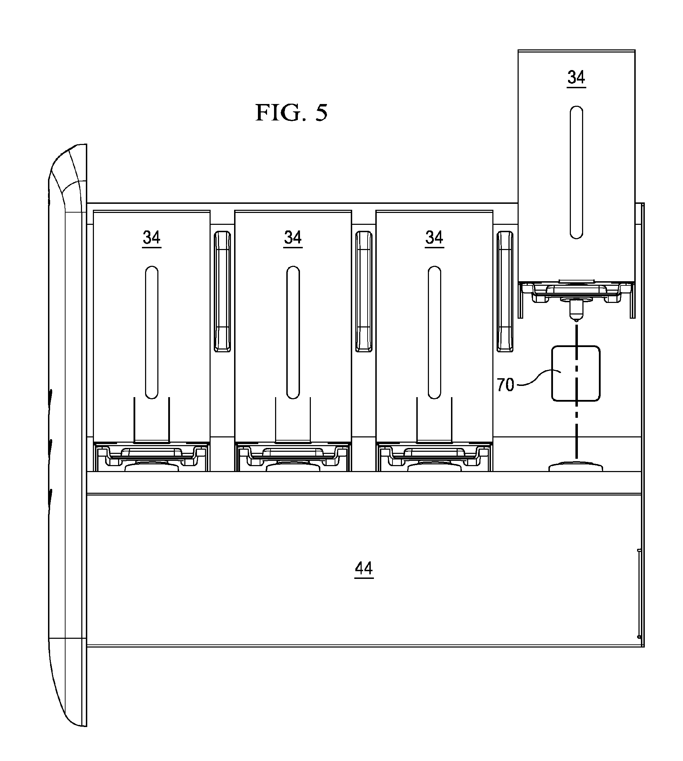

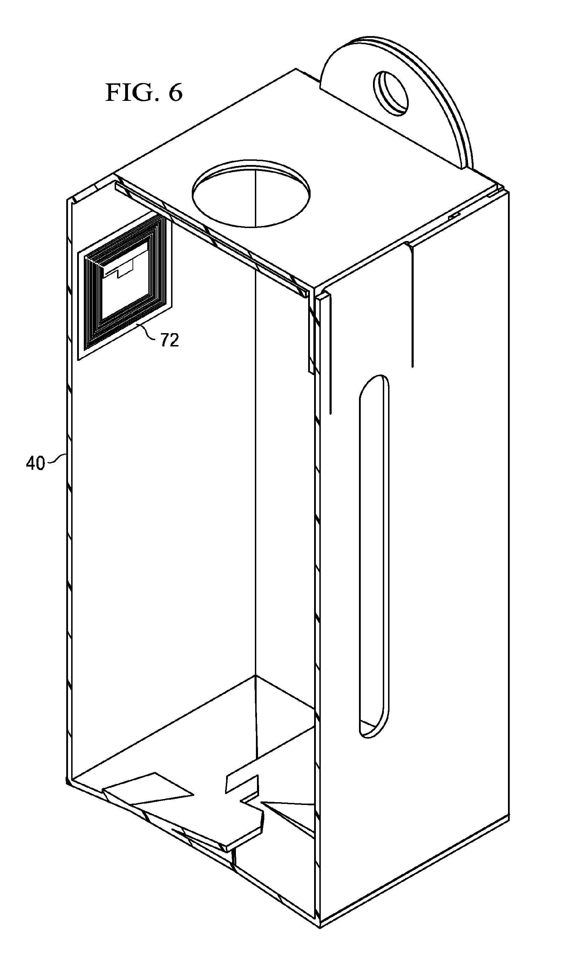

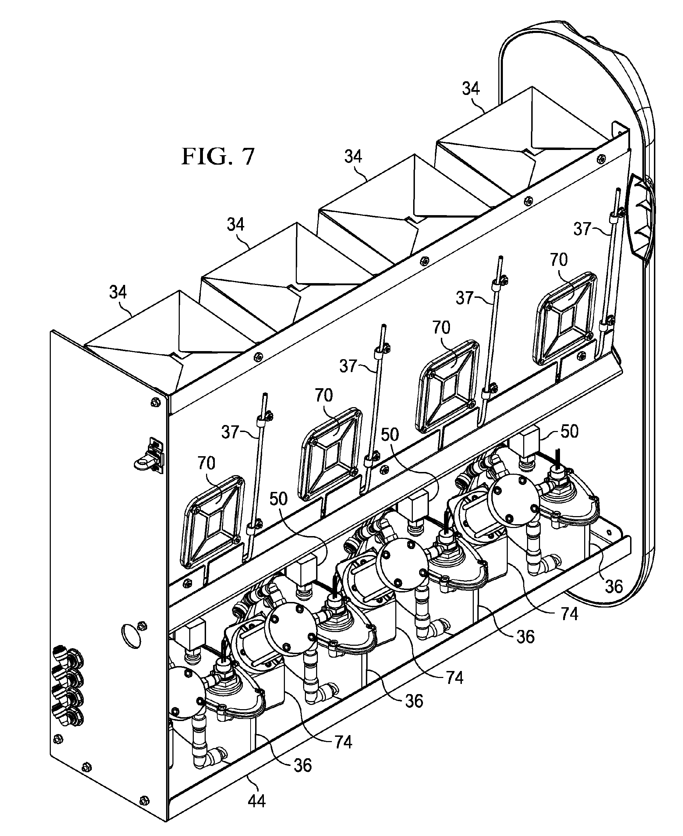

The tanning booth 64 includes a retractable drawer 44 and a touch-screen interface 66. The drawer 44 holds one or more removable containers and allows them to be coupled to the reservoir and vent system 10 described herein. In certain embodiments, the drawer may hold four removable containers, each removable container connected by a coupler 42 to be in fluid communication with a respective reservoir and a pump as shown and described below with respect to Figure 7. Figure 5 shows a detail of the retractable drawer 44 showing three bag-in-box containers 34 positioned within the drawer 44. A fourth container 34 is shown exploded above a corresponding position in the drawer 44. Each position in the drawer 44 includes an interrogator 70. The interrogator 70 receives data communicated from a machine readable tag attached to the removable container 34. For example, as shown in Figure 6, a radio frequency identification (RFID) tag is attached to an inside surface of a wall of the box 40. The bag and a wall of the box have been removed in Figure 6 to more clearly show the position of the tag 72.

In operation, an operator inserts a container 34 into a corresponding position in the drawer 44. Once in position, the RFID tag 72 may be read by the interrogator 70. Accordingly, it is communicated to the controller 25 that a full container has been fluidly coupled to system. The system 10 then performs purging and/or calibration operations specific to that solution. For example, the controller may direct that a purging operation be performed. The controller 25 sends a signal that opens the vent valve 50 and cycles the pump 12 a predetermined number of cycles to clear the conduits and the reservoir 36 of any remaining solution that originated in the depleted container. If purging is not performed (if the depleted solution is replaced with the same type of solution), the presence of the removable container causes the controller request input from the user indicating that the controller is to send a signal to open the vent valve and vent the reservoir such that the liquid from the removable container flows into the reservoir and can be pumped to the nozzles 16. Upon filling the reservoir to a predetermined level as detected by a float sensor, the vent valve 50 closes for normal operation of the system.

In alternate embodiments, any suitable sensor for determining the presence of the removable container may be used. For example, certain embodiments may include a weight sensor to sense the weight of the container 34 in the drawer 44. A suitable sensing system may also include a capacitance sensor or a movement sensor provided the sensor systems are operable to determine the presence of a bag-in-box container 34 in a receiving portion of the drawer 44.

Figure 7 illustrates a specific embodiment of a reservoir and vent system according to the teachings of the present disclosure. Figure 7 shows four pump systems 74, each of which are in fluid communication with a respective reservoir 36. The reservoir 36 is vented and fluidly coupled to the vent tube 37. The vent valve 50 opens and closes the vent.

In addition, the pump 74 and the vent valve 50 are in communication with a respective interrogator 70 through a controller. The interrogator may read information stored on a machine readable tag, such as an RFID tag, attached to a removable container of skin treatment solution, such as a sunless tanning solution. The removable container may contain any type of solution used in the application of a cosmetic to the skin in a spraying operation. For example, the solution may be a solution for pre-tanning, moistening, tanning, and post-tanning. The solution may be clear or bronze and may be water or oil based.

Each of the pump systems 74 may be plumbed to allow solution to be pumped from a respective reservoir 36 to the plurality of spray nozzles 16. In this manner, each pump 74 may be operable to pump a different solution to the spray nozzle 16. Thus, an individual may receive a spray tanning session that includes treatment from a plurality of different skin treatment solutions. For example, the individual may receive a pre-tanning solution in a first treatment operation from a first reservoir 36 pumped by a first pump system 74. The same person, in a second subsequent treatment operation, may receive a bronzer skin treatment solution pumped by a second pump system 74 and sprayed on the individual. After a number of tanning sessions are completed, the liquid level in the reservoir 36 drops, which signals the opening of the vent valve 50 to facilitate continued siphoning from the reservoir and preparation to continue operation with a replacement container.

## Claims

1. A system for spraying a skin treatment solution, comprising:
a reservoir having an inlet and an outlet, the inlet configured to be in fluid communication with a source of a skin treatment solution;
at least one spray nozzle fluidly coupled to the outlet of the reservoir and configured to spray the skin treatment solution; and
a vent valve operable to open and close based on a level of the skin treatment solution in the reservoir, the open vent valve exposing a cavity of the reservoir to at least atmospheric pressure.

2. The system of claim 1, wherein the vent valve is operable to open the vent when the skin treatment solution in the reservoir drops to a predetermined level; and/or wherein the vent valve is operable to close the vent when the skin treatment solution in the reservoir rises to a predetermined level; and/or
wherein the vent valve comprises a solenoid actuator.

3. The system of claim 2, wherein opening the vent valve allows the skin treatment solution to flow from the source to the reservoir.

4. The system according to any one of the claims 1 to 3, further comprising a pump in fluid communication with the reservoir, the pump operable to cause the skin treatment solution in the reservoir to flow to the at least one spray nozzle.

5. The system of claim 4, wherein the vent valve is operable to open when the skin treatment solution in the reservoir drops to a predetermined level and opening the vent valve allows the pump to continue causing the skin treatment solution in the reservoir to flow to the at least one spray nozzle.

6. The system according to any one of the claims 1 to 5, wherein the source of the skin treatment solution is a removable container; preferably wherein the removable container is a bag-in-box container.

7. The system according to any one of the claims 1 to 6, wherein the skin treatment solution is a sunless tanning solution.

8. The system according to any one of the claims 1 to 7, further comprising a vent tube extending upward from the reservoir.

9. A system for spraying a skin treatment solution, according to any one of claims 1 to 8, comprising:
a plurality of reservoirs, each reservoir:
having an inlet and an outlet, the inlet of each reservoir configured to be in fluid communication with a source of a skin treatment solution;
a liquid level sensor operable to detect a level of the skin treatment solution in the reservoir; and
a vent valve operable to open and close based on the detection of the level of the skin treatment solution in the reservoir, the open vent valve exposing a cavity of the reservoir to at least atmospheric pressure; and
at least one spray nozzle fluidly coupled to each outlet of the plurality of reservoirs and configured to spray the skin treatment solution.

10. The system of claim 9, wherein the vent valve is operable to open when the liquid level sensor detects a drop of the level of the skin treatment solution in the reservoir to a predetermined level; and/or
wherein the vent valve is operable to close when the liquid level sensor detects a rise of the level of the skin treatment solution in the reservoir to a predetermined level; and/or
wherein the vent valve is coupled to a vent tube extending upward from the reservoir; and/or
wherein the vent valve comprises a solenoid actuator.

11. The system of claim 9 or 10, wherein opening the vent valve allows the skin treatment solution to flow from the source to the reservoir.

12. The system according to anyone of claims 9 to 11, further comprising a plurality of pumps, each pump fluidly coupled to the outlet of a respective reservoir and operable to cause the skin treatment solution in the respective reservoir to flow to the at least one spray nozzle; preferably
wherein the vent valve is operable to open when the liquid level sensor detects a drop of the level of the skin treatment solution in the reservoir to a predetermined level and opening the vent valve allows the pump to continue causing the skin treatment solution in the reservoir to flow to the at least one spray nozzle.

13. The system according to any one of claims 9 to 12, wherein the liquid level sensor comprises a float sensor and a predetermined level of the skin treatment solution causes the float sensor to generate an electrical signal; preferably further comprising a controller operable to receive the electrical signal from the float sensor and cause the solenoid actuator to actuate based on the received electrical signal.

14. A system for spraying a skin treatment solution according to any of claims 1 to 13, comprising:
a controller operable to control at least one vented reservoir system;
at least one spray nozzle fluidly coupled to the at least one vented reservoir system,
the at least one vented reservoir system, comprising:
at least one reservoir having an inlet and an outlet, the inlet configured to be fluidly coupled to a removable container of a skin treatment solution;
a float sensor operable to detect a level of the skin treatment solution in the at least one reservoir, the float sensor communicating a signal to the controller upon detection of the level of the skin treatment solution;
a vent valve electrically coupled to a solenoid actuator, the controller operable to actuate the solenoid actuator and either open or close the vent valve based on the detection of the level of the skin treatment solution in the reservoir by the float sensor, the open vent valve exposing a cavity of the reservoir to at least atmospheric pressure; and
a pump fluidly coupled to the outlet of the at least one reservoir and the at least one spray nozzle, the pump being operable to cause the skin treatment solution in the reservoir to flow to the at least one nozzle.

15. The system of claim 14 further comprising another vented reservoir system being fluidly coupled to the at least one spray nozzle and being controlled by the controller.
